**Europäisches Patentamt**

**European Patent Office**

**Office européen des brevets**

(11) Numéro de publication: **0 159 223**

**B1**

# FASCICULE DE BREVET EUROPÉEN

(45) Date de publication du fascicule du brevet:
31.05.89

(51) Int. Cl.⁴: **C 12 Q 1/70, C 12 Q 1/68**

(21) Numéro de dépôt: **85400445.4**

(22) Date de dépôt: **07.03.85**

(54) **Réactifs et nécessaires pour le dosage quantitatif d'un acide nucléique viral dans un milieu biologique et procédé de dosage de cet acide nucléique viral.**

(30) Priorité: **07.03.84 FR 8403566**

(43) Date de publication de la demande:
**23.10.85 Bulletin 85/43**

(45) Mention de la délivrance du brevet:
**31.05.89 Bulletin 89/22**

(84) Etats contractants désignés:
**AT BE CH DE GB IT LI LU NL SE**

(73) Titulaire: **INSTITUT PASTEUR, 25/28, rue du Docteur Roux, F-75015 Paris (FR)**
Titulaire: **ABBOTT LABORATORIES, 14th Street and Sheridan Road North St, North Chicago Illinois 60064 (US)**

(72) Inventeur: **Brechot, Christian, 8, rue Boissonnade, F-75014 Paris (FR)**
Inventeur: **Tiollais, Pierre, 16, rue de la Glacière, F-75013 Paris (FR)**
Inventeur: **Scotto, Jacques, 8, rue Quatrefages, F-75005 Paris (FR)**
Inventeur: **Kuhns, Mary, 253 Coventry Circle, Vernon Hills II. 60061 (US)**

(74) Mandataire: **Gutmann, Ernest, S.C. Ernest Gutmann - Yves Plasseraud 67, boulevard Haussmann, F-75008 Paris (FR)**

(56) Documents cité:
**EP-A-0 062 286**
**WO-A-84/01174**
**FR-A-2 444 713**
**FR-A-2 492 985**
**FR-A-2 502 785**
**US-A-4 358 535**

**CHEMICAL ABSTRACTS, vol. 99, no. 5, 1er août 1983, page 286, réf. no. 35635g, Columbus, Ohio, US; SCOTTO, JACQUES et al.: "Detection of hepatitis B virus DNA in serum by a simple spot hybridization technique: comparison with results for other viral markers"**
**CHEMICAL ABSTRACTS, vol. 96, no. 21, 24 mai 1982, page 373, réf. no. 177414w, Columbus, Ohio, US; BERNINGER, MARK et al.: "An assay for the detection of the DNA genome of hepatitis B virus in serum"**

(56) Documents cité: (suite)
**CHEMICAL ABSTRACTS, vol. 100, no. 13, 26 mars 1984, page 322, réf. no. 99481a, Columbus, Ohio, US BIOLOGICAL ABSTRACTS, vol. 73, no. 7, juillet 1982, no. 47576, Philadelphia, US; E.J. GOWANS et al.: "Detection of hepatitis B virus DNA sequences in infected hepatocytes by in situ cytohybridization" HEPATOLOGY (1983), vol. 3(3), pages 279-284**

## Description

L'invention concerne des réactifs standardisés pour le dosage quantitatif d'un acide nucléique viral (ADN ou ARN) éventuellement contenu dans un milieu biologique, plus particulièrement un milieu naturel, des nécessaires comportant de tels réactifs et un procédé de dosage dudit acide nucléique naturel. Dans l'une de ses applications préférées, l'invention concerne le dosage quantitatif, par exemple dans un sérum humain ou de primate de l'ADN viral du virus de l'hépatite B et, plus généralement, des virus infectieux ou virions que ce milieu peut contenir.

A l'heure actuelle, il n'existe guère de méthode de dosage quantitatif simple et direct des particules infectieuses, notamment du virion de l'hépatite virale B. On commence utiliser en routine des sondes d'ADN contenant une séquence caractéristique du génome du virus recherché. Par exemple, il a déjà été proposé d'utiliser des sondes contenant une séquence de DNA-HBV ou tout ou partie du gène S du virus de l'hépatite virale B. Ces sondes permettent, dans les conditions décrites dans la littérature technique, des détections qualitatives de la présence ou non du virus ou du virion, dans des échantillons biologiques susceptibles de les contenir. Ces sondes ne se prêtent cependant guère, dans les conditions sous lesquelles elles ont été, utilisées jusqu'à ce jour, à la réalisation de dosages quantitatifs. Par exemple, lorsque le test d'hybridation est réalisé sur un support, par exemple un support de nitrocellulose, la sonde peut en partie être absorbée ou "traverser" le support en question. Ce phénomène ne nuit pas au caractère qualitatif des détections. Il n'en est pas de même, lorsque l'on veut réaliser des dosages quantitatifs.

Des dosages quantitatifs de certaines séquences d'ADN ont dans des circonstances particulières été réalisés sous microscope électronique. Mais il est clair que de telles techniques ne sauraient être utilisées en routine pour des opérations de détection quantitative de particules virales, notamment en vue du diagnostic. Cette méthode ne peut enfin être mise en oeuvre pour des séries de mesures décalées dans le temps, permettant d'apprécier l'évolution de l'infection.

Pour mémoire, à titre illustratif, deux documents de l'art antérieur sont mentionnés, à savoir:

- la demande de brevet EP.A.62.286 qui concerne un procédé et un kit de diagnostic de la présene du virus HBV, mettant en oeuvre un réactif constitué par une séquence d'ADN cloné, purifié et marqué. Le procédé décrit comprend le dépôt de l'échantillon sur un substrat, l'élimination des protéines et la réaction d'hybridation avec ledit réactif marqué;

- l'article de Chemical Abstracts 10 099 481 concerne un procédé de détection de la présence du virus HBV à partir d'un échantillon dépourvu de protéines, cette détection étant réalisée à l'aide d'un réactif obtenu après purification de l'ADN viral;

- l'article de SCOTTO et al (1983) HEOPATOLOGY, vol. 3, No. 3, pp. 279 - 284, décrit un test d'hybridation sur tache, entre une sonde et un échantillon de sérum contenant l'ADN du virus de l'hépatite B. Ces auteurs font référence à des essais de sensibilité de la méthode décrite avec des dilutions successives d'un sérum positif. SCOTTO et al exposent que, partant d'un sérum très infectieux, ils obtinrent encore un signal d'hybridation positif avec une sonde appropriée, lorsqu' ils répétèrent l'essai avec 50 microlitres de ce même sérum dilué dans un rapport de 6 x $10^{-5}$.

SCOTTO et al ont également décrit des expériences de détection d'un HBV DNA cloné, lorsque celui-ci avait été ajouté à un sérum négatif, également pour tester la sensibilité de leur méthode.

L'invention a pour but de fournir des moyens (réactifs, nécessaires de réactifs et procédés) permettant de tels dosages quantitatifs en particulier, l'invention a pour but de mettre à la disposition du laboratoire et des cliniciens des moyens leur permettant, non seulement de dépister une éventuelle infection virale, par exemple l'hépatite virale B chez des patients déterminés, mais encore de leur permettre d'apprécier rapidement l'évolution de l'infection virale et de vérifier et ajuster en conséquence l'efficacité des traitements antiviraux appropriés.

L'invention a naturellement encore pour but de fournir des moyens de mise en oeuvre aisée et ce en routine.

Le réactif standardisé de référence pour le dosage quantitatif par comparaison d'un ADN viral dans un échantillon de milieu biologique naturel compatible avec le développement du virus correspondant comprenant une concentration déterminée d'un ADN contenant une séquence d'ADN, cloné, hybridable avec le susdit ADN viral, est caractérisé en ce qu'il contient en mélange avec cet ADN cloné, des protéines.

De préférence, l'ADN cloné se présente sous la forme d'un phage cloné contenant une insertion de l'ADN hybridable avec l'ADN viral.

De préférence, les protéines sont originaires d'un milieu biologique semblable au susdit milieu biologique naturel. De préférence, ce réactif contient les ingrédients essentiels du même milieu biologique naturel, voire même est constitué par le même milieu biologique naturel, si ce n'est qu'il était initialement exempt de l'ADN viral à doser de façon quantitative, ce milieu contenant cependant le susdit phage, à la susdite concentration déterminée.

L'association à l'ADN cloné, de préférence à un phage contenant la séquence hybridable, de protéines permet une fixation de l'ADN sur un support du type membrane ou filtre, dans des conditions sensiblement reproductibles pour des conditions relatives déterminées. Ces constatations s'étendent de la même facon a la fixation d'échantillons à tester sur les mêmes supports.

Le procédé de dosage quantitatif de la teneur en un acide nucléique viral déterminé, éventuellement contenu dans un milieu biologique naturel comprend la mise en contact de ce milieu contenant éventuellement cet acide nucléique viral avec une sonde d'ADN, ce milieu ayant été préalablement amené dans des conditions autorisant une telle hybridation, avec une sonde d'ADN, ce procédé étant caractérisé:

- par des mises en contact de plusieurs réactifs distincts contenant respectivement un acide nucléique cloné hybridable avec cette même sonde et préalablement amenés dans des conditions permettant de telles hybridations,

- par la mesure du rapport du résultat relatif à la quantité de sonde hybridée avec l'acide nucléique viral recherché dans le milieu étudié au bruit de fond mesuré dans les mêmes conditions avec un témoin négatif,

- par les mesures obtenues dans des conditions semblables, sinon identiques, respectivement des rapports des résultats relatifs aux quantités de la même sonde hybridée avec les acides nucléiques clonés des susdits réactifs standardisés, au bruit de fond correspondant d'un réactif témoin,

- par le fait que ces réactifs standardisés contiennent ces ADNs clonés en mélange avec une composition de protéines semblables, sinon identiques à celles du milieu biologique et permettant la fixation sur un support du type membrane ou filtre, et par le fait que ces réactifs standardisés se distinguent du réactif témoin par leur teneur en acide nucléique cloné hybridable avec la sonde et entre eux-mêmes par des concentrations déterminées respectivement distinctes en cet acide nucléique cloné, et

- par la comparaison du rapport mesuré avec l'acide nucléique viral recherché dans le milieu biologique étudié avec les rapports obtenus avec les acides nucléiques clonés des susdits réactifs, la teneur en acide nucléique viral du milieu biologique étudié résultant alors d'une corrélation avec les résultats de ces comparaisons.

Le rapport de référence sus-indiqué peut également résulter d'abaques pré-établies à partir des rapports des mesures des quantités de sonde fixées avec des proportions déterminées, respectivement distinctes entre elles de l'ADN cloné, aux bruits de fond correspondants.

Il va de soi que dans ce qui précède le témoin négatif a une composition semblable ou identique celle du réactif, excepté l'absence de l'ADN cloné.

Les quantités de sondes hybridées peuvent être corrélées à tout système mesurable. Par exemple, l'un des partenaires de l'hybridation est porteur d'un marqueur radioactif enzymatique, fluorescent, etc., donnant lieu à la production d'un signal dont l'intensité peut être mesurée.

La quantification possible de l'acide nucléique mis en jeu dans la réaction d'hybridation résulte de la stabilité observée des mesures du susdit rapport, lorsque l'opération d'hybridation entre une sonde appropriée et une quantité prédéterminée du réactif selon l'invention est répétée. Il en va de même pour ce qui est des rapports mesurables dans les mêmes conditions sur un échantillon naturel contenant une dose également toujours semblables (par exemple les parties aliquotes) d'un même échantillon biologique.

Ces rapports sont cependant variables en fonction des quantités d'ADN cloné que peut contenir le réactif utilisé, de sorte qu'il est possible de construire des abaques représentatives de la variation du susdit rapport en fonction des concentrations en ADN cloné, notamment en particules de phage. Les variations du même rapport résultant des mesures semblables effectuées sur des échantillons biologiques testés, sont alors corrélables aux susdites abaques, de sorte que la teneur en particules virales initialement inconnue pour un échantillon biologique déterminé pourra découler d'une simple comparaison des mesures effectués avec la courbe d'une abaque correspondante.

Il résulte de ce qui précède que, lorsque l'échantillon biologique naturel contenant la particule virale à déposer est constituée par un sérum sanguin, il sera avantageux d'utiliser un réactif standardisé conforme à l'invention, dans lequel l'ADN cloné sera associé aux constituants essentiels d'un sérum sanguin. Si l'échantillon à tester est formé à partir d'un extrait cellulaire, les réactifs mis en oeuvre seront avantageusement constitués par un mélange d'un extrait cellulaire équivalent et d'une proportion prédéterminée de l'ADN cloné.

En ce qui concerne l'ADN cloné lui-même, il se présente sous la forme du phage dont le patrimoine génétique a été modifié par insertion de la séquencesonde, par exemple tout ou partie de DNA-HBV. Cette séquence est donc "empaquetée" dans un phage distinct, dont de nombreux exemples ont été décrits dans la littérature. Il s'agit par exemple du phage lambda ou du phage M-13, ou de dérivés ou analogues de ces phages. Le phage utilisé sera quelquefois choisi en fonction de la séquence d'ADN-sonde correspondante en particules virales à doser, eu égard à la taille de l'ADN d'insertion qu'il est capable d'empaqueter.

L'avantage d'utiliser un ADN cloné dans un phage réside dans la similitude entre l'élément cloné et la particule naturelle, de sorte qu'à concentrations voisines en particules virales, on observe des comportements semblables tant du réactif que de l'échantillon dosé vis-à-vis d'une sonde ADN déterminée, lorsque les opérations d'hybridation sont conduites dans des conditions semblables. Ceci étant, il convient de noter que dans certains cas l'ADN cloné pourra être constitué par un plasmide contenant la séquence hybridable avec une partie du génome de la particule virale à doser.

En ce qui concerne la sonde elle-même qui va être hybridée, aussi bien avec l'ADN cloné qu'avec l'acide nucléique viral de l'échantillon à tester, on peut avoir recours à toute sonde connue pour sa capacité à s'hybrider avec l'ADN cloné et le génome de la particule virale recherchée. Dans le cas préféré où les particules virales à doser sont constituées par des virus de l'hépatite B, la sonde pourra être constituée par toute séquence partielle ou entière de DNA-HBV.

Il va de soi que la sonde peut d'ailleurs également être constituée de la même façon que les susdits réactifs, dès lors que leur mise en contact mutuelle peut conduire à une hybridation détectable et mesurable, dans les conditions qui ont été indiquées.

C'est en général la sonde qui sera marquée, soit par un marqueur radioactif, soit par toute autre forme de marqueur, fluorescent ou enzymatique. Dans le cas du marqueur enzymatique, la quantité d'ADN marqués mise en jeu dans l'hybridation, peut être appréciée par le niveau d'action de l'enzyme sur le substrat correspondant, lorsque la modification de ce dernier peut être appréciée quantitativement de toute façon appropriée, notamment par colorimétrie ou spectrophotométrie.

Bien entendu, on peut avoir recours à toute technique classique, pour réaliser la liaison entre la sonde hybride et le marqueur utilisés. Le marqueur peut être préincorporé à la sonde : cela peut être le cas lorsque le marquage est effectué par l'intermédiaire d'un radioélément. Cette liaison peut être directe ou indirecte dans le cas des marqueurs non radioactifs. En particulier, la sonde peut avoir été modifiée pour porter un site antigénique susceptible d'être reconnu par de premiers anticorps, lesquels sont à leur tour reconnus par de seconds anticorps qui portent l'enzyme ou le marqueur fluorescent mis en jeu dans le dosage sus-indiqué.

On se reportera à cet égard avec avantage aux techniques décrites, par exemple, dans le brevet français n° 7 810 975 le brevet américain n° 4 358 535 et le brevet européen n° 00 063 879.

La réaction d'hybridation proprement dite peut être réalisée dans toutes conditions connues appropriées. On peut opérer sur une composition biologique éventuellement pré-enrichie en ADN et obtenue à partir de l'échantillon à étudier. Dans une telle hypothèse, il sera avantageux d'utiliser des réactifs standardisés du type susdéfini, lesquels auront alors avantageusement subi les mêmes types de pré-enrichissement. Cette prépurification ne sera en général pas nécessaire. Le traitement préalable de l'échantillon peut être limité à l'obtention des résultats que constituent la solubilisation des particules virales à détecter et à doser et la dénaturation de l'ADN ou de l'ARN viral.

La réaction d'hybridation peut être réalisée en phase liquide ou et de préférence sur un support solide.

Il est avantageux de réaliser le dépôt, d'une part, de l'échantillon à doser et, d'autre part, le réactif contenant l'ADN cloné sur des supports appropriés aux opérations d'hybridation ultérieures, par exemple sur un filtre de nitrocellulose ou un support de polyamide connu sous la marque NYLON. Ce dépôt est réalisé dans des conditions également connues pour que les séquences d'ADN que contiennent tant l'échantillon que le réactif soient pratiquement fixées sur le support. Par fixation, il faut entendre tout mode de retenue correspondant à une véritable fixation, par exemple par l'intermédiaire de liaisons covalentes, ou encore une simple complexation, voire même adsorption, conduisant cependant au résultat global équivalent à celui d'une fixation. On réalise alors la mise en contact, dans des conditions en elles-mêmes connues, de l'échantillon et du réactif respectivement avec une sonde commune. Le dosage est effectué ultérieurement après lavage adéquat du support pour éliminer toute sonde apportée et excédentaire.

On peut également utiliser des techniques mettant en oeuvre des réactions de compétition. De telles techniques mettent en jeu la compétition à l'égard d'un substrat approprié en quantité déterminée d'un premier constituant apte à réagir avec le substrat, mais en quantité inconnue, et un second constituant, en quantité connue, en général identique ou analogue au premier. La concentration initiale du premier constituant découle alors de la quantité ou de la concentration du second constituant engagé dans la réaction avec le substrat.

Dans le cadre de l'invention, le substrat sera notamment constitué par la sonde, le premier constituant par l'échantillon à doser et le second constituant par un réactif conforme à l'invention.

Il va de soi que l'on aura également dans cette technique établi des abaques permettant la détermination par comparaison des teneurs en la particule virale recherchée de l'échantillon biologique soumis au test.

Pour réaliser des dosages quantitatifs aussi précis que possible, il sera souvent avantageux de disposer d'un nécessaire ou "kit" comportant une pluralité de ces réactifs, ceux-ci se distinguant cependant entre eux par des concentrations en ADN clonés respectivement distinctes. En effet, il peut être avantageux de disposer de suffisamment de réactifs, dans un même nécessaire, pour être toujours à même de mettre en présence avec l'échantillon à doser au moins un réactif dont la teneur en ADN cloné n'est pas trop éloignée de la quantité inconnue à doser pour que les résultats soient les plus significatifs possibles.

L'invention concerne encore des nécessaires ou "kits" caractérisés en ce qu' ils comprennent:
- au moins un réactif tel que décrit ci-dessus et éventuellement
- un réactif de même composition, si ce n'est qu'il est dépourvu de l'ADN cloné.

Il comprend avantageusement en outre au moins une abaque représentative des variations observables du rapport des intensités mesurees d'un signal fourni par un marqueur, après hybridation avec la sonde de différentes concentrations du réactif selon, l'invention au bruit de fond mesurée dans des conditions semblables par contact du réactif témoin avec la même sonde et dans des conditions d'hybridation identiques, en fonction des proportions de réactifs mises en jeu dans ces essais d'hybridation, étant entendu que le marqueur est porté par l'un ou l'autre des partenaires mis en jeu dans l'hybridation, et de préférence par la sonde.

Le nécessaire comprend de préférence en outre un ou plusieurs supports appropriés au dépôt et à la fixation sur un support des différents ingrédients des réactifs selon l'invention du réactif témoin et de l'échantillon à tester, dans des conditions permettant leur hybridation ultérieure éventuelle avec une sonde d'ADN contenant également une séquence hybridable.

D'autres caractéristiques de l'invention apparaîtront encore au cours de la description qui suit de modes de préparation préférés des réactifs standardisés ainsi que de leur mise en oeuvre, de l'établissement d'une abaque pouvant être utilisée comme référence pour des dosages quantitatifs ultérieurs et d'un mode de mise en oeuvre du procédé selon l'invention.

1°) Préparation des échantillons

Des échantillons de sérums sanguins présumés être infectés par le virus de l'hépatite a sont fixés sur une membrane du, polyamide nylon (Biodine A, Pall Co) ou sur tout autre support de nylon ou de nitrocellulose approprié pour des essais d'hybridation. Les dépôts sont effectués en taches "Dot-blots". Ces dépôts peuvent être réalisés soit par dépôts directs des échantillons concentrés, soit par filtration des échantillons préalablement dilués à travers des zones localisées du support et par aspiration à travers ces zones. Les échantillons sont ensuite traités avec une solution de soude 0,15 M et de NaCl 1 M (concentrations finales) pour solubiliser sur place les particules virales et dénaturer les ADN et les rendre accessibles à une sonde susceptible de s'hybrider au génome du virus HBV.

On neutralise ensuite avec un tampon Tris: NaCl. La phase solide est ensuite portée à 80°C et la cuisson est maintenue pour une durée de 1 à 2 heures, selon la nature du support utilisé, cuisson dont l'effet est alors d'assurer la fixation des ADN de l'échantillon sur le support.

Les échantillons sont alors prêts pour la réalisation des opérations ultérieures d'hybridation avec des réactifs standardisés conformes à l'invention.

2°) Préparation des réactifs standardisés

Les ADN clonés mis en oeuvre dans ces réactifs sont constitués par des phages tels que les phages lambda ou M-13, dont les génomes ont été modifiés dans une région non essentielle pour la réplication de ces phages par une insertion d'un ADN ayant une séquence commune avec le génome des particules virales à détecter, par exemple une séquence partielle ou complète de DNA-HBV, séquence alors empaquetée dans les phages en question.

Un ou plusieurs réactifs conformes à l'invention sont préparés par mélange de ces phages modifiés en concentrations déterminées (concentrations déterminées par exemple par des mesures de densités optiques ou par des plaques de lyse de cultures cellulaires en tapis) avec du sérum sanguin sain. Il est avantageux de réaliser des réactifs de ce type permettant la mise en oeuvre de doses unitaires de particules s'étageant de $10^2$ à $10^{10}$ particules de phages.

Ces réactifs sont alors déposés également sur filtre et traités dans les mêmes conditions que les échantillons à doser. Les membranes comprenant finalement des dépôts d'ADNs en quantités connues (taches formées par les réactifs) et en quantités inconnues (taches obtenues à partir des échantillons à doser) sont prêtes pour la réalisation de l'hybridation dans des conditions classiques, par exemple avec une sonde DNA-HBV marquée par un élément radioactif, par exemple $^{32}p$.

On a recours aux techniques d'hybridation classiques, à la suite de quoi les supports sont lavés pour éliminer la sonde non fixée. Les dosages sont ensuite faits directement sur le filtre (autoradiogrammes) ou, par exemple, par scintillographie en milieu, après découpe des taches et introduction de celles-ci dans des cuves de mesures contenant un liquide scintillateur approprié.

3°) Préparation d'une abaque caractéristique

Une membrane-filtre de nitrocellulose est posée à la surface d'une plaque comportant une pluralité de trous, elle-même posée sur une cuve reliée à une pompe aspirante permettant la réalisation d'une dépression légère s'exerçant sur la membrane au niveau des trous (diamètre 12 mm) de la plaque. La membrane est humidifiée avec un tampon SSC et dans les puits de profondeur limitée que forment alors les parties de la membrane au-dessus des trous de la plaque, on dépose, dans les conditions qui ont été indiquées plus haut, des doses croissantes du réactif de l'invention contenant des particules, par exemple de DNA-HBV empaquetées dans un phage lambda, en suspension dans du sérum sanguin, pour obtenir une série de dépôts comportant respectivement des quantités de particules s'étageant entre $10^2$ et $10^{10}$ particules virales par puits. On réalise le contact dans des conditions permettant l'hybridation avec une sonde marquée, par exemple au $^{32}p$, comme il a été indiqué. Après les étapes de préhybridation et d'hybridation réalisées dans des conditions classiques, on mesure, pour chaque puits, le niveau de radioactivité retenu et on établit le rapport de la radioactivé mesurée au bruit de fond (établi avec un échantillon témoin traité dans les mêmes conditions, mais exempt de particules virales soumises aux mêmes opérations de dosage) et l'on trace la courbe correspondant à la variation des rapports radioactivité mesurée/bruit de fond, en fonction des nombres de particules virales contenus dans les réactifs antérieurement introduits dans les puits.

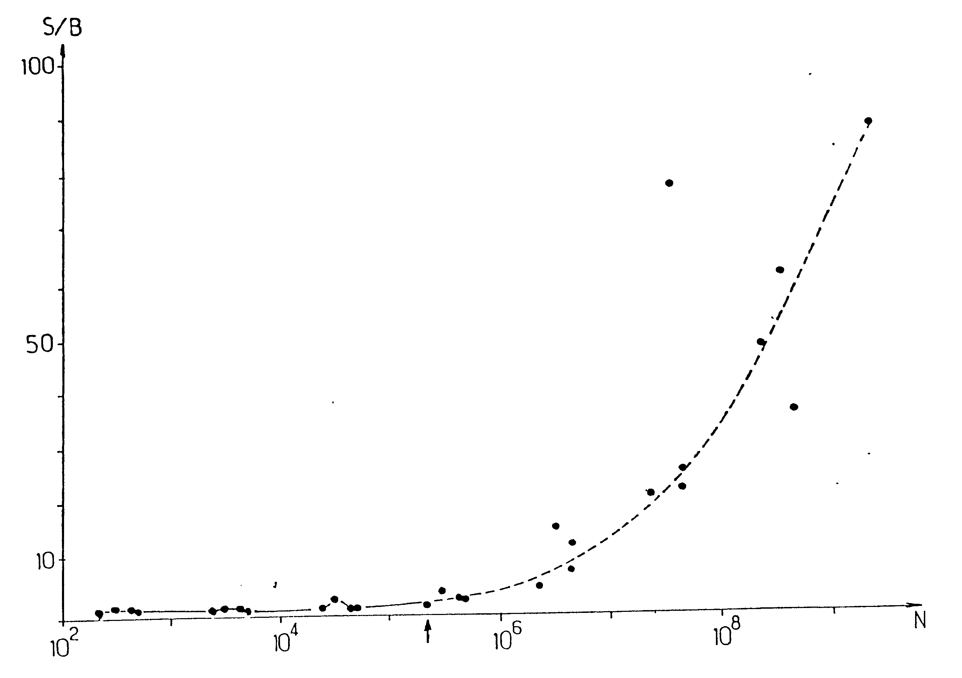

On obtient alors la courbe représentative des variations de ce rapport (S/B: abréviation de "signal/bruit") en fonction des concentrations particulaires initialement mises en oeuvre. Une telle abaque résulte, par exemple, de la figure unique jointe. Elle illustre la variation observée du rapport 5/B (sur l'axe des ordonnées) en fonction des proportions initiales déterminées de particules du phage déposées dans chaque puits (en nombre N de particules par puits sur l'axe des abscisses). Les mesures ont été faites directement sur le filtre par autoradiogramme.

S'agissant maintenant de déterminer la teneur en particules virales d'un échantillon à étudier, on mesure et on détermine, dans les mêmes conditions que précédemment indiqué, le rapport S/B. La détermination de la teneur inconnue en particules virales découle alors de la comparaison du rapport S/B obtenu et de l'abaque, en tenant compte s'il a lieu d'un facteur de correction, également prédéterminé, éventuellement dû à l'éventuelle différence de composition entre les réactifs mis en oeuvre et les échantillons à tester.

On peut tracer des abaques du même type pour tous autres types de réactifs contenant des concentrations en ADN clonés empaquetés distincts.

Pour des mesures quantitatives, il y aura lieu de retenir comme crédibles les valeurs des mesures correspondant à des rapports S/B supérieurs à 2.

Une méthode quantitative de dosage qui peut être utilisée en variante vise des séquences d'HBV-DNA marquées radioactivement et d'activité spécifique connue, immobilisés sur un support solide. Ces réactifs standards immobilisés sont alors traités, comme indiqué ci-dessus. On réalise une hybridation entre ces séquences immobilisées et un jeu de réactifs radioactivement marqués de concentrations connues en particulles de phages, d'une part et un second jeu de doses déterminées d'un échantillon à doser dont le contenu en particules virales naturelles est inconnu.

La quantité d'ADN finale retenue sur le support solide peut alors être calculée et l'on peut déterminer aussi bien l'efficacité d'hybridation que le dosage quantitatif des particules hybridées provenant des échantillons à doser.

D'une façon générale, la méthode dont un exemple de mise en oeuvre vient d'être donné peut être adaptée à tous autres types de particules virales et à toutes sondes correspondantes mettant en jeu des marqueurs différents, par exemple des marqueurs enzymatiques ou fluorescents. La méthode peut être utilisée pour la détermination de concentrations relatives de tout ADN ou ARN particulier d'origine virale dans des fluides biologiques, tissus ou cellules. Les réactifs selon l'invention sont également applicables à la production d'étalons internationaux, tant en ce qui concerne les réactifs que des échantillons contenant les particules virales naturelles correspondantes.

Le procédé selon l'invention fournit par conséquent une méthode de dosage quantitatif direct de particules, par exemple de particules d'HBV, dont la mise en oeuvre est particulièrement simple. Elle est particulièrement utile lorsqu'il s'agit de déterminer le niveau d'infection dont souffre un patient, pour l'appréciation de l'évolution de l'infection et de l'évaluation de l'efficacité d'un traitement antiviral.

Comme il va de soi et comme il résulte d'ailleurs déjà de ce qui précède, l'invention ne se limite nullement à ceux de ses modes d'application et de réalisation qui ont été plus spécialement envisagés elle en embrasse au contraire toutes les variantes.

**Revendications**

1. Procédé de dosage quantitatif de la teneur en un acide nucléique viral déterminé, éventuellement contenu dans un milieu biologique naturel, comprenant la mise en contact de ce milieu contenant éventuellement cet acide nucléique viral avec une sonde d'ADN, ce milieu ayant été préalablement amené dans des conditions autorisant une telle hybridation, avec une sonde d'ADN, caractérisé:

- par des mises en contact de plusieurs réactifs distincts contenant respectivement un acide nucléique cloné hybridable avec cette même sonde et préalablement amenés dans des condition permettant de telles hybridations,

- par la mesure du rapport du résultat relatif à la quantité de sonde hybridée avec l'acide nucléique viral recherché dans le milieu étudié au bruit de fond mesuré dans les mêmes conditions avec un témoin négatif,

- par les mesures obtenues dans des conditions semblables, sinon identiques, respectivement des rapports des résultats relatifs aux quantités de la même sonde hybridée avec les acides nucléiques clonés des susdits réactifs standardisés, au bruit de fond correspondant d'un réactif témoin,

- par le fait que ces réactifs standardisés contiennent ces ADNs clonés en mélange avec une composition de protéines semblables, sinon identiques à celles du milieu biologique et permettant la fixation sur un support du type membrane ou filtre, et par le fait que ces reactifs standardisés se distinguent du réactif témoin par leur teneur en acide nucléique cloné hybridable avec la sonde et entre eux-mêmes par des concentrations déterminées respectivement distinctes en cet acide nucléique cloné, et

- par la comparaison du rapport mesuré avec l'acide nucléique viral recherché dans le milieu biologique étudié avec les rapports obtenus avec les acides nucléiques clonés des susdits réactifs, la teneur en acide nucléique viral du milieu biologique étudié résultant alors d'une corrélation avec les résultats de ces comparaisons.

2. Procédé selon la revendication 1, caractérisé en ce que les réactifs standardisés contiennent les ingrédients essentiels du milieu biologique naturel, voire sont constitués par le même milieu biologique naturel que celui contenant l'acide nucléique viral à doser, si ce n'est que ce milieu était initialement exempt de l'ADN viral à doser, ces réactifs contenant cependant en outre le susdit ADN cloné en des concentrations déterminées respectivement distinctes.

3. Procédé selon la revendication 2, caractérisé en ce que les réactifs consistent en un sérum sanguin initialement sain contenant cet ADN cloné en des concentrations respectivement distinctes.

4. Procédé selon l'une quelconque des revendications 1 à 3, caractérisé en ce que l'acide nucléique viral déterminé à doser est constitué par un ADN du virus de l'hépatite B et en ce que l'ADN cloné de chacun des réactifs contient, une séquence d'ADN hybridable avec le génome du virus de l'hépatite B, tel que DNA-HBV ou le gène 5 du génome du virus de l'hépatite B.

5. Procédé selon l'une quelconque des revendications 1 à 4 dans lequel l'ADN cloné de chacun des réactifs standardisés est constitué par un phage cloné dans lequel est insérée une séquence d'ADN hybridable avec la susdite sonde.

6. Procédé selon l'une quelconque des revendications 1 à 5, caractérisé en ce que l'ADN cloné de chacun des réactifs standardisés comporte un marqueur.

7. Procédé selon l'une quelconque des revendications à 6, caractérisé en ce que chacun des réactifs standardisés est fixé sur un support dans des conditions autorisant l'hybridation ultérieure de son ADN cloné avec la sonde d'ADN capable également d'hybrider avec l'acide nucléique viral à doser.

8. Procédé selon l'une quelconque des revendications 1 à 7, caractérisé en ce que les rapports de référence se rapportant aux réactifs standardisés résultent d'une abaque pré-établie à partir des mesures effectuées dans des essais d'hybridation de ces réactifs avec la sonde, cette abaque étant alors représentative de la variation des rapports du genre en question en fonction des concentrations de l'acide nucléique hybridé contenues dans lesdits réactifs, et en ce que le rapport mesuré pour l'acide nucléique viral à doser dans le milieu est comparé avec les rapports de référence découlant de l'abaque.

9. Nécessaire ou "kit" pour le dosage quantitatif par comparaison d'un acide nucléique viral dans un milieu biologique naturel compatible avec le développement du virus correspondant, conformément au procédé selon l'une quelconque des revendications 1 à 8, comprenant:

- une sonde d'hybridation avec l'acide nucléique viral à doser,
- un réactif témoin, formé d'une composition de protéines semblables ou équivalentes à celles dudit milieu biologique, et permettant la fixation d'un acide nucléique sur un support du type membrane ou filtre, si ce n'est que ce réactif témoin est dépourvu de l'ADN cloné,
- plusieurs réactifs standardisés de référence qui contiennent la composition de protéines du réactif témoin et comprennent des ADNs clonés, contenant une séquence d'ADN hybridable avec la susdite sonde d'hybridation.

10. Nécessaire selon la revendication 9, caractérisé en ce qu'il comprend à la fois des susdits réactifs fixés sur un support et des susdits réactifs non fixés sur un support, les réactifs fixés ne comportant pas de marqueurs et les réactifs non fixés portant un marqueur.

11. Nécessaire selon la revendication 9 ou la revendication 10, caractérisé en ce qu'il comprend en outre au moins une abaque représentative des variations du rapport des intensités mesurées d'un signal fourni par un marqueur, après hybridation entre la sonde et les ADNs clonés des différents réactifs, au bruit de fond mesuré dans des conditions semblables par contact du réactif témoin avec la même sonde et (dans des conditions d'hybridation identiques, ces variations étant fonction des proportions d'ADN cloné contenues dans les réactifs mis en jeu dans ces essais d'hybridation, étant entendu que le marqueur est porté par l'un ou l'autre des partenaires mis en jeu dans l'hybridation, et de préférence par la sonde.

12. Nécessaire selon l'une' quelconque des revendications 9 à 11, caractérisé en ce que les ADNs clonés des réactifs de référence contiennent une séquence hybridable avec le génom du virus de l'hépatite virale B, notamment DNA-HBV, ou le gène S du génome du virus de l'hépatite virale B.

**Patentansprüche**

1. Verfahren zur quantitativen Bestimmung des Gehalts an einer bestimmten viralen Nukleinsäure, die gegebenenfalls in einem nattürlichen biologischen Medium enthalten ist, umfassend das Zusammenbringen dieses Mediums, das gegebenenfalls diese virale Nukleinsäure enthält, mit einer DNS-Sonde, wobei das Medium vorläufig auf solche Bedingungen eingestellt worden ist, die einederartige Hybridbildung mit einer DNS-Sonde erlauben, gekennzeichnet

- durch das Zusammenbringen mehrerer unterschiedlicher Reagentien, die jeweils eine geklonte, mit der gleichen Sonde hybridisierbare Nukleinsäure enthalten, und zuvor Einstellen auf Bedingungen, die diese Hybridisierungen erlauben;

- durch Messung des Verhältnisses des Ergebnisses, relativ zur Menge, deren Sonde mit der untersuchten viralen Nukleinsäure hybridisiert in dem untersuchten Medium, zum Grundrauschen, gemessen unter den gleichen Bedingungen mit einem negativen Vergleich,

- durch die unter ähnlichen, wenn nicht identischen Bedingungen erhaltenen Messungen bzw. Verhältnisse der Ergebnisse, relativ zu den Mengen der gleichen mit den geklonten Nukleinsäuren der oben erwähnten standardisierten Reagentien hybridisierten Sonde, zum Grundrauschen, das einem Vergleichsreagens entspricht;

- durch die Tatsache, daß diese standardisierten Reagentien diese geklonten DNS im Gemisch mit einer Zusammensetzung aus Proteinen enthalten, die ähnlichen, wenn nicht identisch zu denen des biologischen Mediums sind und die Fixierung auf einem Träger vom Membran- oder Filtertyp erlauben, und durch die Tatsache, daß diese standardisierten Reagentien sich von dem Vergleichsreagens durch ihren Gehalt an geklonter Nukleinsäure, die mit der Sonde und untereinander hybridisierbar sind, durch die bestimmten bzw. unterschiedlichen Konzentrationen an dieser geklonten Nukleinsäure unterscheiden, und

- durch Vergleich des gemessenen Verhältnisses der untersuchten viralen Nukleinsäure in dem untersuchten biologischen Medium mit den Verhältnissen, die mit den mit den oben erwähnten Reagentien geklonten Nukleinsäuren erhalten werden, wobei sich der Gehalt an viraler Nukleinsäure in dem untersuchten biologischen Medium in Korrelation zu den Ergebnissen der Vergleiche ergibt.

2. Verfahren nach Anspruch 1,
dadurch gekennzeichnet, daß die standardisierten Reagentien die wesentlichen Bestandteile des natürlichen biologischen Medium enthalten oder sogar aus dem gleichen natürlichen biologischen Medium bestehen, das die zu bestimmende virale Nukleinsäure enthält, wenn nicht das Medium von Anfang an frei von der zu bestimmenden viralen DNS ist, wobei jedoch die Reagentien außerdem die oben erwähnte geklonte DNS in bestimmten bzw. unterschiedlichen Konzentrationen enthalten.

3. Verfahren nach Anspruch 2,
dadurch gekennzeichnet daß die Reagentien aus einem zunächst gesunden Blutserum bestehen, die die geklonte DNS in bestimmten bzw. unterschiedlichen Konzentrationen enthalten.

4. Verfahren nach einem der Ansprüche 1 bis 3,
dadurch gekennzeichnet daß die bestimmte, quantitativ zu bestimmende virale Nukleinsäure aus einer DNS des Hepatitis-B-Virus besteht und daß die mit jedem der Reagentien geklonte DNS eine mit dem Genom von Hepatitis-B-Virus hybridisierbare DNS-Sequenz, wie DNS-HBV, oder das Gen S des Genoms des Heptatis-B-Virus enthält.

5. Verfahren nach einem der Ansprüche 1 bis 4, dadurch gekennzeichnet, daß die mit jedem der standardisierten Reagentien geklonte DNS aus einem geklonten Phagen besteht, in den eine mit der oben erwähnten Sonde hybridisierbare DNS-Sequenz insertiert ist.

6. Verfahren nach einem der Ansprüche 1 bis 5, dadurch gekennzeichnet daß die geklonte DNS jedes der standardisierten Reagentien eine Markierung enthält.

7. Verfahren nach einem der Ansprüche 1 bis 6, dadurch gekennzeichnet daß jedes der standardisierten Reagentien auf einem Träger unter Bedingungen fixiert ist, die die spätere Hybridisierung seiner geklonten DNS mit der DNS-Sonde erlauben, die gleichfalls mit der zu bestimmenden viralen Nukleinsäure hybridiseren kann.

8. Verfahren nach einem der Ansprüche 1 bis 7, dadurch gekennzeichnet daß die Referenzverhältnisse, die sich auf standardisierte Reagentien beziehen, aus einem Nomogramm erhalten worden sind, das, ausgehend von Messungen, die bei Hybridisierungsuntersuchungen dieser Reagentien mit der Sonde durchgeführt worden sind, vorher aufgestellt worden ist, wobei dieses Nomogramm repräsentativ für die Variation der Verhältnisse der fraglichen Art als Funktion der Konzentrationen der in diesen Reagentien enthaltenen hybridisierten Nukleinsäure ist, und daß das in dem Medium für die zu bestimmende virale Nukleinsäure gemessene Verhältnis mit den sich aus dem Nomogramm ergebenden Referenzverhältnissen verglichen wird.

9. Analyseneinheit oder "kit" für die quantitative Bestimmung durch Vergleich einer viralen Nukleinsäure in einem natürlichen biologischen Medium, das mit der Entwicklung des entsprechenden Virus verträglich ist, entsprechend dem Verfahren nach einem der Ansprüche 1 bis 8, welche(s) umfaßt:

- eine Sonde für die Hybridisierung mit der zu bestimmenden viralen Nukleinsäure;

- ein Vergleichsreagens, gebildet aus einer Zusammensetzung aus Proteinen, die ähnlich oder entsprechend zu denen des biologischen Mediums sind, das die Fixierung einer Nukleinsäure auf einem Träger vom Membran- oder Filtertyp erlaubt, wenn es nicht das Vergleichsreagens ohne die geklonte DNS ist;

- mehrere standardisierte Bezugsreagentien, die die Proteinzusammensetzung des Vergleichsreagens enthalten und die geklonten DNS umfassen, und die eine mit der oben erwähnten Hybridisierungssonde hybridisierbare DNS-Sequenz enthalten.

10. Analyseneinheit nach Anspruch 9,
dadurch gekennzeichnet daß sie gleichzeitig die oben erwähnten, auf einem Träger fixierten Reagentien und die oben erwähnten nicht auf einem Träger fixierten Reagentien enthält, wobei die fixierten Reagentien keine Markierung enthalten und die nicht fixierten Reagentien eine Markierung enthalten.

11. Analyseneinheit nach Anspruch 9 oder Anspruch 10, dadurch gekennzeichnet daß sie außerdem mindestens ein Nomogramm enthält, das die Veränderungen des Verhältnisses der gemessenen Intensitäten eines Signals, das durch eine Markierung nach Hybridisierung mit den geklonten DNS-Sonden erzeugt wird zum Grundrauschen, das unter ähnlichen Bedingungen bei Berührung des Vergleichsragens mit der gleichen Sonde und unter identischen Hybridisierungsbedingungen gemessen wird, zeigt wobei diese Veränderungen eine Funktion des Anteils an geklonter DNS ist, die in den für die Hybridisierungsuntersuchung eingesetzten Reagentien enthalten ist, wobei die Markierung natürlich an dem einen oder anderen für die Hybridisierung eingesetzten Reaktionsteilnehmer und vorzugsweise an der Sonde vorhanden ist.

12. Analyseneinheit nach einem der Ansprüche 9 bis 11, dadurch gekennzeichnet daß die geklonten DNS der Referenzreagentien eine Sequenz enthalten, die mit dem Genom des Hepatitis-B virus insbesondere DNS-HBV, oder dem Gen S des Genoms des Hepatitis-B Virus.

## Claims

1. Method of quantitative assay of the content of a predetermined viral nucleic acid, possibly contained in a natural biological medium, comprising the contacting of this medium containing possibly this viral nucleic acid with a DNA probe, this medium having previously been brought under conditions permitting such hybridation, with a DNA probe, characterised:
- by the contacting of several distinct reagents respectively containing a cloned nucleic acid, hybridable with said probe and previously brought under conditions permitting such hybridations,
- by measuring the ratio of the result relating to the amount of probe hybridised with the viral nucleic acid sought in the studied medium, to the background noise measured under the same conditions with a negative control,
- by the measurement obtained under similar conditions, if not identical, respectively of the ratios of the results relating to the quantities of the same probe hybridised with the cloned nucleic acids of the abovesaid standardised reagents, to the corresponding background noise of the control reagent,
- by the fact that these standardised reagents contain these cloned DNAS in admixture with a composition of proteins similar if not identical, to those of the biological medium and enabling the fixation to a support of the membrane or filter type and by the fact that these standardised reagents distinguishes of the control reagent by their content of cloned nucleic acid hybridable with the probe and from one-another, by determined concentrations of this cloned nucleic acid respectively distinct, and

- by the comparison of the ratio measured with the viral nucleic acid sought in the biological medium studied, with the ratios obtained with the cloned nucleic acids of said reagents, the content of viral nucleic acid of the biological medium studied, resulting then from a correlation with the results of the comparison.

2. Method according to claim 1 characterised in that the standardised reagents contain the essential ingredients of the natural biological medium, even are constituted of the same natural biological medium as that containing the viral nucleic acid to be quantitatively assayed, except that this medium was initially free of the viral DNA to be quantitatively assayed, these reagents containing however in addition the above cloned DNA in determined concentrations respectively distinct.

3. Method according to claim 1 characterised in that the reagents consist of a blood serum initially healthy containing this cloned DNA in respectively distinct concentrations.

4. Method according to anyone of claims 1 to 3, characterised in that the determined viral nucleic acid to be quantitatively assayed is constituted by a DNA of the hepatitis B virus and in that the cloned DNA of each of the reagents contains a DNA sequence hybridable with the genome of the hepatitis B virus, such as DNA-HBV or the S gène of the genome of the hepatitis B virus.

5. Method according to anyone of claims 1 to 4 wherein the cloned DNA of each of the standardised reagents is constituted by a cloned phage in which a DNA sequence, hybridable with the above probe, is inserted.

6. Method according to anyone of claims 1 to 5, characterised in that the cloned DNA of each of the standardised reagents comprises a marker.

7. Method according to anyone of claims 1 to 6, characterised in that each of the standardised reagents is fixed on a support, under conditions enabling subsequent hybridation of its cloned DNA with the DNA probe, also capable of hybridation with the nucleic acid to be quantitatively assayed.

8. Method according to anyone of claims 1 to 7, characterised in that the reference ratios related to the standardised reagents result from a nomogram preestablished from the measurements performed in hybridation assays of these reagents with the probe, this nomogram being then representative of the variation of said ratios as a function of the concentrations of the hybridated nucleic acid contained in said reagents, and in that the measured ratio for the viral nucleic acid to be quantitatively assayed in the medium is compared with the reference ratios resulting from the nomogram.

9. Outfit or "kit" for the quantitative assay of a viral DNA in a natural biological medium compatible with the developement of the corresponding virus, according to the process of anyone of claims 1 to 8, comprising:
- a probe hybridable with the viral nucleic acid to be quantitatively assayed,

- a control reagent, formed by a composition of proteins similar or equivalent to those of said biological medium and enabling the fixation of a nucleic acid on a support of the membrane or filter type, except that the control reagent is free of the cloned DNA,

- several standardised reference reagents which contain the composition of proteins of the control reagent and comprise cloned DNAs, containing a DNA sequence hybridable with said hybridation probe.

10. Outfit according to claim 9, characterised in that it comprises both the abovesaid reagents fixed to a support and the abovesaid reagents not fixed to a support, said fixed reagents not including a marker and said unfixed reagents bearing a marker.

11. Outfit according to claim 9 or to claim 10, characterised in that it comprises in addition at least one nomogram representative of the variations in the ratio of the measured intensities of a signal provided by a marker, after hybridation between the probe and the cloned DNAs of the different reagents, to the background noise measured under similar conditions by contact of the control reagent with the same probe and under identical hybridation conditions, these variations being a function of proportions of cloned DNA contained in the reagents employed in these hybridation tests, it being understood that the marker is borne by one or other of the partners employed in the hybridation, and preferably by the probe.

12. Outfit according to anyone of claims 9 to 11, characterised in that the cloned DNAs of the reference reagents contain a sequence hybridable with the genome of the hepatitis B virus, particularly DNA-HBV, or the S gene of the genome of the hepatitis B virus.